# EUROPÄISCHE PATENTSCHRIFT

(11) **EP 1 588 693 B1**
(45) Veröffentlichungstag und Bekanntmachung des Hinweises auf die Patenterteilung: **06.06.2007**
(21) Anmeldenummer: 05007232.1
(22) Anmeldetag: 02.04.2005
(51) Int. Cl.: A61K 8/37, A61Q 17/04

(54) **Verbesserte Lösungsvermittler/Lösungsmittel für organische UV-Filter**
Improved solubiliser/solvent for organic uv-filters
Agent de solubilisation/solvant amélioré pour les filtres uv

(30) Priorität: 14.04.2004 DE 102004018510
(43) Veröffentlichungstag der Anmeldung: 26.10.2005
(73) Patentinhaber: Goldschmidt GmbH, 45127 Essen (DE)
(72) Erfinder: Grüning, Burghard, Dr., 45134 Essen (DE); Grzebyk, Pawel, 46147 Oberhausen (DE); Jenni, Klaus, Dr., 45357 Essen (DE); Schunicht, Christoph, Dr., 45147 Essen (DE)

(56) Entgegenhaltungen:
- EP-A- 0 950 398
- WO-A-00/47544
- FUNAHSHI K: "New ring opening reactions of oxiranes with aryl carboxylates" BULLETIN OF THE CHEMICAL SOCIETY OF JAPAN, NIHON KAGAKKAI, TOKYO, JP, Bd. 52, Nr. 5, 1979, Seiten 1488-1492, XP002109078 ISSN: 0009-2673

## Beschreibung

Die vorliegende Erfindung betrifft verbesserte Lösungsvermittler/Lösungsmittel für organische UV-Filter und die Verwendung dieser Materialien in kosmetisch akzeptablen Produkten zum verbesserten Schutz gegen die UV-Strahlung sowie kosmetische Formulierungen, die den Anwender besser gegen W-Strahlung schützen können.

Es ist seit langem bekannt, dass insbesondere der ultraviolette Anteil von natürlichen und künstlichen Lichtquellen (UV-A 320 bis 390 nm; UV-B 280 bis 320 nm; UV-C 100 oder 200 bis 280 nm) in größeren Mengen zur Schädigung der menschlichen Haut führt.

Die UV-A-Strahlung bewirkt hauptsächlich die Hautalterung (Oberhautverdünnung und Bindegewebedegeneration, Pigmentstörungen) während UV-B und UV-C zu Sonnenbrand und Hautkrebs führen.

Das insbesondere in den letzen Jahren veränderte Freizeitverhalten mit verlängerten Aufenthalten im Freien und insbesondere extensives Sonnenbaden zur Erzielung der "gesunden Bräune" haben allerdings vor dem Hintergrund der medizinischen Erkenntnisse, des Bewußtseins der mangelnden natürlichen Schutzmechanismen der Haut durch Pigmentbildung und Sonnengewöhnung durch Verdickung der Hornschicht, die Notwendigkeit eines ausreichenden Schutzes vor intensiver UV-Strahlung weit in den Vordergrund gerückt. Deutlich verstärkt wurde sie durch die Diskussion der Abnahme und Verdünnung des antarktischen Ozonloches und der.damit einhergehenden Steigerung der Intensität der UV-A- und W-B-Strahlung auf der Erdoberfläche.

Das wird deutlich an den in den letzten Jahren steigenden Umsätzen von Produkten mit hohen Sonnenschutzfaktoren (sun protection factor: SPF). Dies sind in erster Linie immer noch die klassischen Sonnenschutzformulierungen (Sonnenmilch, Sonnenöl) mit dem primären Anwendungszweck Sonnenbaden, jedoch zunehmend auch die sogenannten Pflegeprodukte für Gesicht, Körper und Haar wie Tages- und Nachtcremes, Conditioner, Lotionen, (Hydro-, Lipo-)Gelen, (Lippen-)Stiften und Sprays, pharmazeutische Formulierungen und in geringem Umfang Produkte der dekorativen Kosmetik, die überwiegend in Form von Ölen und flüssigen, cremeförmigen oder salben/pastenartigen W/O- und O/W-Emulsionen im Handel sind.

Wie oben dargelegt, kann die UV-B-Strahlung eher Bräunung und Verbrennungen hervorrufen, als die UV-A-Strahlung. Daher enthielten die Sonnenschutzmittel überwiegend nur Filter, die gegen die UV-B-Strahlung schützen. Da die Wirkungen/Nebenwirkungen der Sonnenbräune/des Sonnenbrands nicht sofort deutlich wahrnehmbar werden, wird die Haut deutlich länger der Strahlung ausgesetzt als es angebracht wäre.

Damit ist die Haut jedoch vorwiegend der UV-A-Strahlung ungeschützt ausgesetzt. Das Problem besteht nun darin, dass die UV-A-Strahlung in die Haut eindringt und einen Langzeitschaden verursacht, auch wenn sie nicht sofort erkennbare Wirkungen hervorruft.

Durch medizinische Erkenntnisse der letzten Jahre wurde klar belegt, dass nicht nur die UV-B-Strahlung, sondern auch die UV-A-Strahlung schädlich für die Haut ist. Es wurde belegt, dass Enzyme, die durch die UV-B-Strahlung geschädigte Zellen reparieren, in ihrer Aktivität gehemmt werden und somit die UV-A-Strahlung Hautkrebs indirekt fördert. Durch die höhere Eindringtiefe kann die UV-A-Strahlung darüber hinaus Veränderungen in Blutgefäßen verursachen.

Des Weiteren stellen sie den Ursprung der meisten Photodermatosen dar, wie Sonnenallergien: kleine rote Bläschen, die bei der ersten Sonnenexposition auf Armen oder Dekolleté erscheinen und von starkem Juckreiz begleitet werden. Auf lange Sicht können sie bei Missbrauch der Sonne Hautkrebs hervorrufen.

Die UV-A-Strahlen sind daher fast gefährlicher als die UV-B-Strahlen, da kein Alarmsignal wie der Sonnenbrand von ihnen ausgeht. Wenn man ihre Schäden bemerkt, ist es bereits zu spät.

Bei erhöhter Sonnenexposition, hat man einen Anstieg verschiedener infektiöser Hautkrankheiten wie Mykosen oder Herpes festgestellt.

Dieser Anstieg beruht auf der Fähigkeit der UV-Strahlen, das Immunsystem der Haut zu schwächen, so ihr Reaktionsvermögen zu vermindern und die Abwehr gegen Auslöser von Infektionen wie beispielsweise den Herpesvirus oder Hautpilze zu verringern. Es wird heute angenommen, dass das Phänomen der Photoimmunosuppression auch in der Entstehung von Hautkrebs eine bedeutende Rolle spielt.

Für den Sonnenschutz der bloßen, unbedeckten Haut und der Haare (Bleichung, Versprödung), insbesondere aber des Gesichts und der Lippen, sind daher Sonnenschutzmittel gefragt, die ausreichenden und anhaltenden Schutz über das gesamte schädigende UV-Spektrum gewähren.

Solche Breitband-Sonnenschutzmittel können eine Kombination entsprechender organischer UV-A und UV-B Filter enthalten.

Hierunter sind bei Raumtemperatur flüssig oder kristallin vorliegende organische Substanzen zu verstehen, die in der Lage sind, ultraviolette Strahlen zu absorbieren und die aufgenommene Energie in Form längerwelliger Strahlung, z. B. Wärme wieder abzugeben. Zur Einstellung eines ausreichend hohen Sonnenschutzfaktors müssen allerdings entsprechend hohe Anteile an diesen Filtern verwendet werden.

Bei anhaltender Sonnenexposition der Haut sollte der Schutz in regelmäßigen Abständen von ca. ein bis vier Stunden erneuert werden. Gleiches gilt bei sportlicher Aktivität, um die Abnahme des Schutzes, d. h. Verlust der Filtersubstanzen durch Baden, Schwitzen oder mechanischen Abrieb durch Kleider oder Handtuch, auszugleichen.

Nur die regelmäßige Verwendung von Lichtschutzprodukten mit einem hohen SPF und einem breiten Absorptionsspektrum sowohl gegen UVA- als auch UVB-Strahlen erlaubt einen wirksamen Schutz. Darüber hinaus werden vermehrt aufgrund der in manchen Regionen oftmals erhöhten Sonneneinstrahlung Sonnenschutzformulierungen verlangt, die einen stark erhöhten Lichtschutzfaktor besitzen.

Der Lichtschutzfaktor LSF oder auch SPF ist ein Koeffizient, welcher das Vermögen eines Produktes ausdrückt, Sonnenbrand durch die Sonne zu verhindern. Lichtschutz mit einem Faktor von 60 schützt daher doppelt so lange wie ein Produkt mit Faktor 30 und entsprechend 3 mal solange wie ein Produkt mit Faktor 20 vor dem Auftreten eines Sonnenbrandes.

Diese höheren Lichtschutzfaktoren werden in den meisten Fällen erzeugt durch eine Erhöhung der Konzentration von UV-Filtersubstanzen in der Formulierung.

Seit 1995 werden die Lichtschutzfaktoren nach derselben internationalen Norm (COLIPA) gemessen, die einen Vergleich zwischen den verschiedenen Herstellern erlaubt.

Bei diesen häufigen und großflächigen Anwendungen ist es nicht ausgeschlossen, dass die hochdosierten Filter (ca. 3 bis 30 Gew.-% der Formulierung) in Gramm-Mengen auf die Haut aufgetragen werden.

Diese Mengen an Filtersubstanzen müssen aber gelöst und homogen und stabil in der Formulierung eingearbeitet worden sein.

Zum Lösen dieser Substanzen wird häufig von ölartigen Komponenten Gebrauch gemacht, die ein gutes Lösungsvermögen für die Filtersubstanzen besitzen. So werden unter anderem auch bestimmte Esteröle eingesetzt. Eine verwendete Verbindungsklasse sind hier aliphatische Benzoesäureester. Ein typischer Vertreter dieser Verbindungsklasse ist die Verbindung Tegosoft® TN (C₁₂₋₁₅-Alkylbenzoat), welche besonders häufig als Lösungsmittel für UV-Filtersubstanzen eingesetzt wurde.

Allerdings reicht das Lösungsvermögen der etablierten Verbindungen oftmals nicht aus, um größere Mengen von UV-Filtersubstanzen zu lösen.

Somit ist diese Konzentrationserhöhung in der Praxis oftmals problematisch oder unter Umständen sogar unmöglich.

Darüber hinaus können unvollständig gelöste UV-Filteranteile im Endprodukt unter Umständen den angegebenen SPF in Frage stellen. Selbst bei anfänglich sich noch vollständig darstellenden Lösungseigenschaften des Sonnenschutzfilters kann es dennoch bei extremen, aber praxisrelevanten Lagerbedingungen zu einem Ausfallen der Filter und somit zu einem Wirkungsverlust kommen.

Aufgabe der vorliegenden Erfindung war es, diese Nachteile zu überwinden und kosmetische Formulierungen mit einem besonders hohen Lösungsvermögen für UV-Filter zur Verfügung zu stellen.

Diese Aufgabe wird gelöst durch die Verwendung von neuen Lösungsvermittlern/Lösungsmitteln zur Lösung von organischen UV-Filtern.
Die Verbindungen zeichnen sich durch ihr besonders hohes Lösungsvermögen für organische kristalline UV-A- und UV-B-Filter aus.
Gleichzeitig weisen die Verbindungen einen vergleichsweise hohen Anteil an aromatischen Gruppen im Molekül auf.
Aus dem Stand der Technik ist nun bekannt, dass die Einführung von aromatischen Gruppen wie z.B. Phenylresten in ein Molekülgerüst oftmals eine deutliche Erhöhung des Schmelzpunktes zur Folge hat, so dass die Verbindungen bei Raumtemperatur fest vorliegen. Teilweise weisen Verbindungen mit einem hohen aromatischen Anteil Schmelzpunkte von > 100 °C auf, wodurch die Einarbeitung in kosmetische Formulierungen stark erschwert ist. Überraschenderweise sind die erfindungsgemäßen Verbindungen trotz ihres vergleichsweise hohen Anteils an aromatischen Gruppen bei Raumtemperatur flüssig oder besitzen einen niedrigen Schmelzpunkt von < 70 °C.
Durch ihren niedrigen Schmelzpunkt können die erfindungsgemäßen Substanzen bei den in der Kosmetikindustrie bevorzugten niedrigen Verarbeitungstemperaturen unterhalb des Siedepunktes von Wasser in kosmetische Formulierungen eingearbeitet werden. Darüberhinaus können die erfindungsgemäßen Verbindungen, die bei Raumtemperatur fest vorliegen, vorteilhaft in Kombination mit flüssig vorliegenden Verbindungen eingesetzt werden, wobei flüssige Mischungen erhalten werden können.

Diese Eigenschaften machen die erfindungsgemäßen Verbindungen zu geeigneten Inhaltsstoffen von kosmetischen Formulierungen.

Ein Gegenstand der Erfindung ist daher die Verwendung von mindestens einer der Verbindungen der Formeln 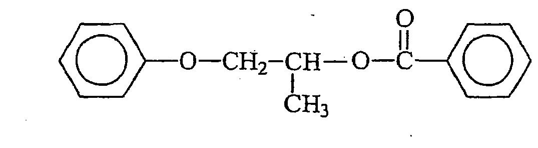 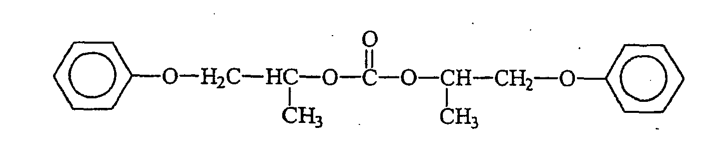 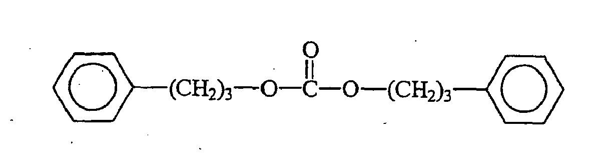 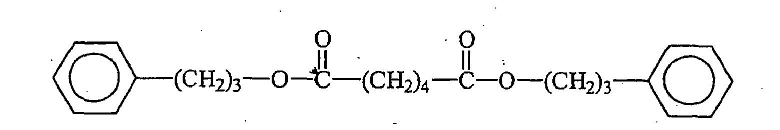 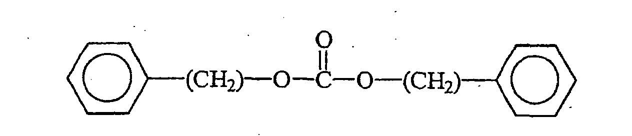 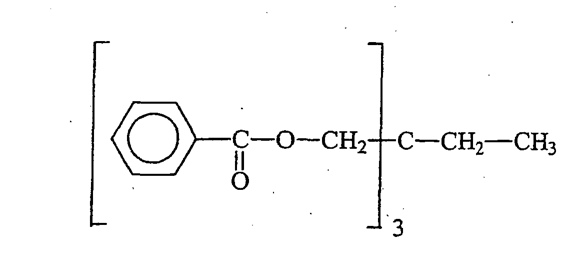 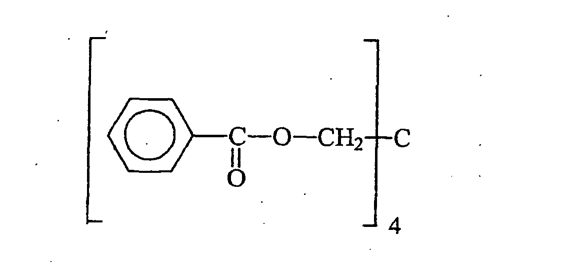 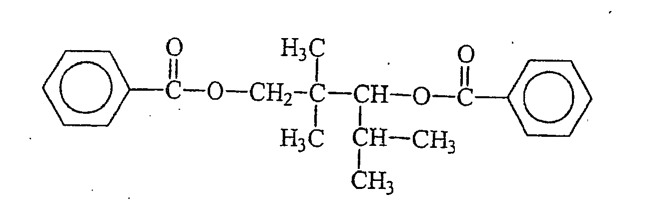 als Lösungsvermittler/Lösungsmittel zur Lösung von organischen UV-Filtern.

Ein weiterer Gegenstand der Erfindung ist die Verwendung dieser Lösungsvermittler/Lösungsmittel zur Lösung von organischen UV-Filtern für die Herstellung kosmetischer Formulierungen.

Ein weiterer Gegenstand der Erfindung sind kosmetische Formulierungen mit verbessertem Sonnenschutzfaktor enthaltend organische UV-Filter gelöst in den verbesserten Lösungsvermittlern/Lösungsmitteln.

Erfindungsgemäß mitverwendbare organische UV-Filter sind beispielsweise die nachfolgend aufgeführten Verbindungen (FDA-Registration Name in Klammern) :

| Filter | FDA-Registration Name |
|---|---|
| Ethylhexyl-Methoxycinnamate | Octinoxate |
| Benzophenone-3 | Oxybenzone |
| | Octocrylene |
| Butyl-Methoxydibenzoylmethane | Avobenzone |
| Ethylhexyl-Salicylate | Octisalate |
| Homobornyl-Salicylate | Homosalate |
| Phenylbenzimidazole-Sulfonic-Acid | Phenylbenzimidazole-Sulfonic-Acid |
| Benzophenone-4, -5 | Sulisobenzone |
| Ethylhexyl-Dimethyl-PABA | Padimate O |
| 4-Aminobenzoic-Acid | PABA |
| Butyl-Methoxycinnamate | Cinoxate |
| Benzophenone-8 | Dioxybenzone |
| Menthyl-Anthranilate | Menthyl-Anthranilate |
| 4-Methylbenzylidene-Camphor | |
| Ethylhexyl-Triazone | |
| PEG-25-PABA | |
| Isoamyl-p-Methoxycinnamate | |
| Diethylhexyl-Butamido-Triazone | |
| Drometrizole-Trisiloxane | |
| Camphor-Benzalkonium-Methosulfate | |
| Terephthalylidene-Dicamphor-Sulfonic-Acid | |
| Benzylidene-Camphor-Sulfonic-Acid | |
| 3-Benzylidene-Camphor | |
| Diethylbenzylidene-Malonate-Dimethicone | |
| Methylene-Bis-Benzotriazolyl-Tetramethylbutylphenol | |
| Bis-Ethylhexyloxyphenol-Methoxyphenyl-Triazine | |
| Disodium-Phenyl-Dibenzimidazole-Tetrasulfonate | |
| Polyacrylamidomethyl-Benzylidene-Camphor | |

Die am weitesten verbreiteten chemischen Sonnenschutzmittel enthalten beispielsweise p-Aminobenzoesäure (PABA), PABA-Ester (Glyceryl-PABA, Amyldimethyl-PABA und Octyldimethyl-PABA), Benzophenonderivate (Oxybenzon und Sulisobenzon), Cinnamate (Octylmethoxycinnamat und Cinoxate), Salicylate (Homobornylethylsalicylat) und Anthranilate (siehe beispielsweise Pathak, Madhu, "Sunscreens: Topical and Systemic Approaches for Protection of Human Skin Against Harmful Effects of Solar Radiation", Continuing Medical Education Series, J. Am. Acad. Dermat, 7: 3 (September 1982) S. 285, 291).

Zum Test des Lösungsvermögen von kristallinen UV-Filtern in den beschriebenen Substanzen wurden stellvertretend drei repräsentative UV-A- bzw. UV-B-Filter ausgewählt. Hierbei handelt es sich um Benzophenon-3 (2-Hydroxy-4-methoxy-benzophenon) und Butyl-Methoxydibenzoylmethane als zwei UV-A-Filtern und Methylbenzylidene-Camphor als einem UV-B-Filter.

Das Lösungsvermögen von herkömmlichen Esterölen für diese drei Verbindungen ist in den meisten Fällen nicht befriedigend. Eine Verbindung mit überdurchschnittlichem Lösungsvermögen für UV-Filtersubstanzen ist das schon erwähnte Tegosoft® TN, welches deshalb als Inhaltsstoff für Sonnenschutzformulierungen auch weithin etabliert ist.

Es konnte nun gezeigt werden, dass das Lösungsvermögen der erfindungsgemäßen Verbindungen demjenigen von Tegosoft® TN oftmals nicht nur vergleichbar ist, sondern dieses in vielen Fällen auch deutlich übertrifft.

Auf diese Weise lassen sich die geforderten kosmetischen Formulierungen mit einem besonders hohen Lösungsvermögen für UV-Filter gut realisieren.

Geeignete Träger zur Herstellung derartiger Sonnenschutzformulierungen umfassen Lanolin, Glycerylstearat, Kakaobutter, Sorbitansesquioleat, Propylenglykol, Isopropylmyristat, Petrolatum und Acrylpolymere. Es können ferner Gemische von zwei oder mehreren dieser Stoffe verwendet werden. Diese Stoffe sind im Stand der Technik als "dermatologisch geeignet" bekannt, d.h. sie verursachen oder begünstigen keine nachteiligen Reaktionen auf der Haut des Anwenders.

Die Menge des Trägers muß nur ausreichend sein, um beim Auftragen auf die Haut eine gleichmäßige Verteilung zu erzielen, damit eine ausreichende Abdeckung der Haut mit dem UV-absorbierenden Material sichergestellt ist.

Die oben beschriebenen, öligen Zubereitungen können allein oder in Form von Wasser in Öl (W/O)-Emulsionen in topischen Sonnenschutzmitteln eingearbeitet werden, ferner in verschiedenartige kosmetische Produkte, wie beispielsweise Lippenstift, Lidschatten, Make-up, Feuchtigkeitscreme, Rouge und weitere Pflegeprodukte eingebracht werden, um Kosmetika zu bilden, die die darunterliegende Haut des Anwenders gegen die schädlichen Wirkungen der UV-Strahlung schützen. Diese Materialien können mit bekannten Mischmethoden mit der kosmetischen Grundmasse vermengt werden.

Weitere Bestandteile kosmetischer Formulierungen, die die erfindungsgemäßen Verbindungen enthalten, können sogenannte Hilfsstoffe sein. Diese Hilfsstoffe sind im Stand der Technik wohlbekannt und werden zugegeben, um die ihnen eigenen Funktionen zu erfüllen. Die bevorzugten Hilfsstoffe schließen Stoffe wie Verdickungsmittel, Weichmacher, Überfettungsmittel, Mittel für die Wasserfestigkeit, Emollients, Netzmittel und oberflächenaktive Stoffe sowie Konservierunqsmittel, Antischaummittel, Parfums und deren Gemische oder beliebige weitere kompatible Inhaltsstoffe, die herkömmlich in der Kosmetik verwendet werden, ein.

### Technischer Teil:

Herstellung der erfindungsgemäßen Verbindungen:
Die erfindungsgemäßen Verbindungen sind auf einfachem Wege zu erhalten durch die Veresterung von geeigneten substituierten Carbonsäuren mit den entsprechend geeigneten Alkoholen oder durch Umesterung von Carbonsäureestern mit geeigneten Alkoholen.
Hierbei werden Mono- und Dicarbonsäuren bzw. ihre Ester mit aromatisch substituierten Alkoholen oder aromatisch substituierten Carbonsäuren bzw. ihre Ester mit verzweigten und unverzweigten, wahlweise ebenfalls aromatischen Mono- und Polyalkoholen umgesetzt.
Alternativ können statt der Carbonsäureester auch organische Carbonate wie Diethylcarbonat verwendet werden. Die Herstellung erfolgt nach den üblichen literaturbekannten Vorgehensweisen für Veresterungs- bzw. Umesterungsreaktionen.

### Beispiel 1

### Herstellung von Verbindung 1:

379,9 g des Alkohols 1-Phenoxy-2-propanol werden zusammen mit 320,1 g Benzoesäure, 4,2 g p-Toluolsulfonsäure und 0,7 g Unterphosphoriger Säure vorgelegt. Nachdem auf 160 °C aufgeheizt wurde, legt man ein Vakuum von 400 mbar an. Nach 1,5 h wird das Vakuum auf 200 mbar erhöht und in weiteren Schritten auf 30 mbar erhöht. Anschließend wird die Temperatur auf 180 °C erhöht. Nachdem die Säurezahl einen Wert von < 15 erreicht hat, wird mit Kalilauge neutalisiert und das Produkt einer Wasserdampfbehandlung unterworfen. Anschließend wird das Produkt im Vakuum bei 150 °C getrocknet. Nach dem Abkühlen wird das Produkt filtriert.

Das Produkt liegt als leicht gelbliche klare Flüssigkeit vor mit einem Reinheitsgrad von 90 % (GC). Die Ausbeute beträgt 95 % bezogen auf den Alkohol.

### Beispiel 2

### Herstellung von Verbindung 2:

129,7 g des Alkohols 1-Phenoxy-2-propanol werden zusammen mit 50,3 g Diethylcarbonat vorgelegt. Nachdem auf 110 °C aufgeheizt wurde, werden 0,9 g Isopropyltitanat zugegeben. Nun wird langsam bis auf 240 °C aufgeheizt und das entstehende Ethanol über eine Destillationskolonne abdestilliert.

Nach 4 h wird auf ca. 160 °C abgekühlt und ein Vakuum von 100 mbar angelegt. Das Vakuum wird im Verlauf von 2,5 h auf 10 mbar abgesenkt und das weiter entstehende Ethanol abdestilliert, bis kein weiteres Destillat mehr entsteht. Das Produkt wird einer Wasserdampfbehandlung unterworfen und anschließend bei 150 °C im Vakuum getrocknet. Nach dem Abkühlen wird das Produkt filtriert.
Das Produkt liegt als klare Flüssigkeit vor mit einem Reinheitsgrad von 89 % (GC). Die Ausbeute beträgt 93 % bezogen auf den Alkohol.

Analog zu diesen Beispielen erfolgt die Herstellung der Verbindungen 3 bis 8.

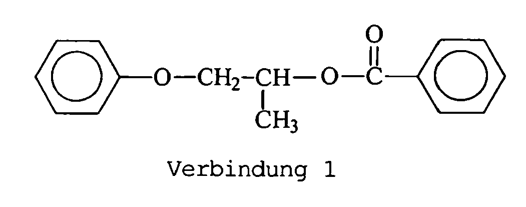

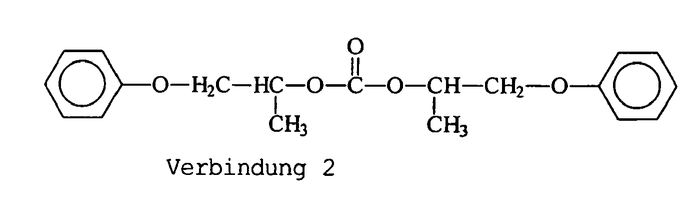

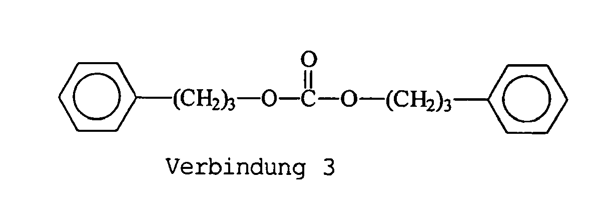

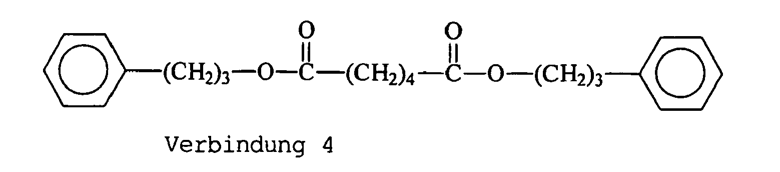

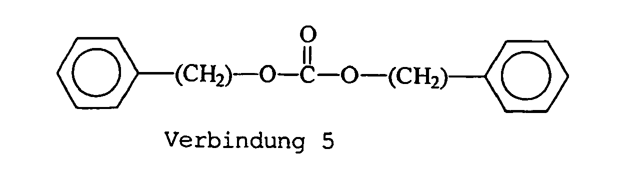

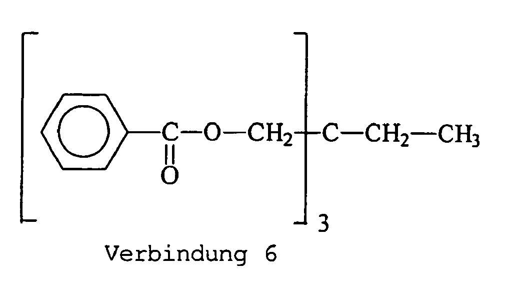

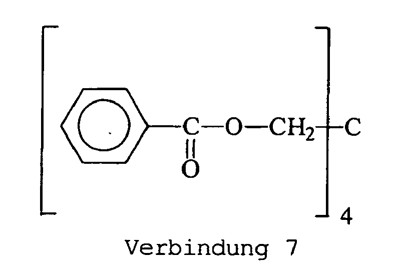

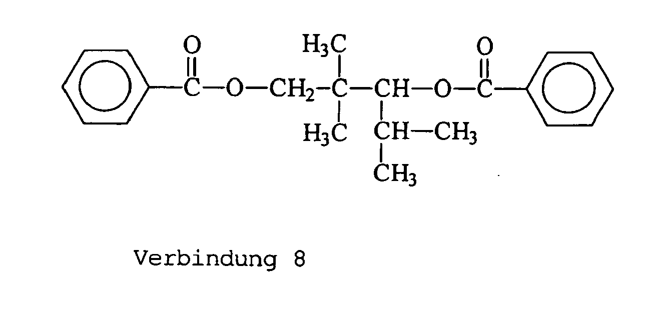

### Test des Lösungsvermögens von kristallinen UV-Filtern:

Zur Bestimmung der Löslichkeit von UV-Filtersubstanzen wurden stellvertretend drei repräsentative kristalline UV-A- bzw. UV-B-Filter ausgewählt. Hierbei handelt es sich um:
Benzophenon-3 (= 2-Hydroxy-4-methoxy-benzophenon) (UV-A-Filter)
4-Methylbenzyliden-campher (UV-B-Filter)
Butyl-methoxy-dibenzoylmethan (UV-A-Filter)

Zur Bestimmung des Lösungsvermögens für diese drei UV-Filtersubstanzen wird jeweils eine bestimmte Menge (50 g) einer der erfindungsgemäßen Verbindungen vorgelegt und auf 22 °C temperiert. Nun wird 1 Gew.-% eines UV-Filters zugegeben und gerührt, bis sich diese Menge vollständig und homogen gelöst hat. Dieser Vorgang wird wiederholt, bis die maximal lösbare Menge des UV-Filters überschritten wurde. Zum vollständigen Auflösen ist bei höheren Konzentrationen oftmals eine längere Rührzeit von mehreren Stunden erforderlich.

Ist auf diese Weise die Maximalkonzentration grob bestimmt worden, wird zur Feinbestimmung der Konzentrationsbereich um diese Maximalkonzentration herum mit kleineren Einwaagemengen des UV-Filters wiederholt.

Als Referenz wurde die Verbindung Tegosoft® TN verwendet.

### Ergebnisse der Löslichkeitsbestimmungen:

| Verbindung | Benzophenon-3 | 4-Methylbenzyliden-campher | Butyl-methoxydibenzoylmethan |
|---|---|---|---|
| 1 | 31 | 30,7 | 16,5 |
| 2 | 19,7 | 24,3 | 9,5 |
| 3 | 18,5 | 23,5 | 9,0 |
| 4 | 25,0 | 23,9 | 9,5 |
| 5 | 29,3 | 27,0 | 8,8 |
| 8 | 24,5 | 29,4 | 15,6 |
| Tegosoft^{®} TN | 12,7 | 22,0 | 12,1 |

Wie aus den obigen Werten ersichtlich, ist das Lösungsvermögen der erfindungsgemäßen Verbindungen in vielen Fällen deutlich besser als das Lösungsvermögen von Tegosoft^{®} TN.

### Beispiele für kosmetische Formulierungen:

Die erfindungsgemäßen Verbindungen wurden als Bestandteil von kosmetischen Formulierungen eingesetzt. Hierzu wurden exemplarisch eine Wasser-in-Öl- (W/O)- bzw. eine Öl-in-Wasser (O/W)-Creme ausgewählt.

### A) Standardprüfrezeptur W/O (W/O-Sonnencreme)

### Herstellung:

Ansatzgröße: jeweils 100 g
1. Bestandteile der Phase A in einem Becherglas bei 80 °C aufschmelzen und in ein Becherglas überführen.
2. Bestandteile der Phase A in einem Trockenschrank bei 80 °C temperieren.
3. Bestandteile der Phase B in einem Becherglas lösen.
4. Phase B langsam unter Rühren mit einem MIG-Rührer (500 U/min) einrühren und anschließend 3 Minuten bei 1.400 U/min homogenisieren.

### Standardprüfrezeptur W/O 1:

Mit Tegosoft^{®} TN als Lösungsmittel für den UV-Filter Benzophenon-3 (12 % bezogen auf das Gemisch mit Tegosoft^{®} TN).

| Standardprüfrezeptur W/O | | |
|---|---|---|
| | | % |
| **A** | ISOLAN^{®} PDI | 3,0 |
| | Castor Wachs | 0,5 |
| | Microwachs W 80 | 0,5 |
| | Tegosoft^{®} TN | 22,85 |
| | Benzophenon-3 | 3,15 |
| | (Gesamt Ölphase 30 %) | |
| | | |
| **B** | MgSO₄*7H₂O | 1,0 |
| | Wasser | 68,95 |
| | Bronopol | 0,05 |

### Standardprüfrezeptur W/O 2:

Mit Verbindung 1 als Lösungsmittel für den UV-Filter Benzophenon-3 (12 % bezogen auf das Gemisch mit Verbindung 1).

Diese Standardprüfrezeptur dient zur Überprüfung, ob sich der Rezepturbestandteil Tegosoft^{®} TN in Standardprüfrezeptur W/O 1 problemlos gegen eine vergleichbare Menge von Verbindung 1 austauschen lässt, ohne die Eigenschaften der Standardprüfrezeptur grundlegend zu verändern.

| Standardprüfrezeptur W/O | | |
|---|---|---|
| | | % |
| **A** | ISOLAN^{®} PDI | 3,0 |
| | Castor Wachs | 0,5 |
| | Microwachs W 80 | 0,5 |
| | Verbindung 1 | 22,85 |
| | Benzophenon-3 | 3,15 |
| | (Gesamt Ölphase 30 % | |
| | | |
| **B** | NaCl | 1,0 |
| | Wasser | 68,95 |
| | Bronopol | 0,05 |

### Standardprüfrezeptur W/O 3:

Mit Verbindung 1 als Lösungsmittel für den UV-Filter Benzophenon-3 (24 % bezogen auf das Gemisch mit Verbindung 1).

Diese Standardprüfrezeptur dient zur Überprüfung, ob sich der Anteil des UV-Filters Benzophenon-3 in der Standardprüfrezeptur gegenüber Standardprüfrezeptur W/O 2 deutlich erhöhen lässt, ohne die Eigenschaften der Standardprüfrezeptur grundlegend zu verändern.

| Standardprüfrezeptur W/O | | |
|---|---|---|
| | | % |
| **A** | ISOLAN^{®} PDI | 3,0 |
| | Castor Wachs | 0,5 |
| | Microwachs W 80 | 0,5 |
| | Verbindung 1 | 19,76 |
| | Benzophenon-3 | 6,24 |
| | (Gesamt Ölphase 30 %) | |
| | | |
| **B** | NaCl | 1,0 |
| | Wasser | 68,95 |
| | Bronopol | 0,05 |

Wie aus der Herstellung und Überprüfung der oben angegebenen Rezepturen ersichtlich wurde, kann sowohl der Rezepturbestandteil Tegosoft^{®} TN in Standardprüfrezeptur W/O 1 problemlos gegen eine vergleichbare Menge von Verbindung 1 ausgetauscht werden und auch der Anteil des UV-Filters Benzophenon-3 in der Standardprüfrezeptur deutlich erhöht werden, ohne die Eigenschaften der Standardprüfrezeptur grundlegend zu verändern.

### B) Standardprüfrezeptur O/W (O/W-Sonnencreme)

### Herstellung:

Ansatzgröße: jeweils 100 g
1. Bestandteile der Phase A in einem 400 ml Becherglas auf 80 °C aufheizen.
2. Bestandteile der Phase B in einem 250 ml Becherglas auf 80 °C aufheizen.
3. Phase B zu Phase A geben und anschließend mit dem ESG-Stab für 2 Minuten homogenisieren.
4. Im Wasserbad unter rühren auf 50 bis 60 °C abkühlen, Phase C zugeben und erneut 1 Minute homogenisieren
5. Im Wasserbad unter rühren auf < 30 °C abkühlen.

### Standardprüfrezeptur O/W 1:

Mit Tegosoft^{®} TN als Lösungsmittel für den UV-Filter Methylbenzylidene-Camphor (20 % bezogen auf das Gemisch mit Tegosoft^{®} TN).

| Standardprüfrezeptur O/W | | |
|---|---|---|
| | | % |
| **A** | TEGO^{®} Care 215 | 2,5 |
| | TEGIN^{®} M | 1,0 |
| | TEGO^{®} Alkanol 18 | 2,0 |
| | TEGOSOFT^{®} TN | 11,6 |
| | Methylbenzyliden-campher | 2,9 |
| | (Gesamt. Ölphase 20 %) | |
| | | |
| **B** | Glycerin | 3,0 |
| | Wasser | 76,5 |
| | CA 24^{®} | 0,1 |
| | | |
| **C** | Keltrol F^{®} | 0,4. |

### Standardprüfrezeptur O/W 2:

Mit Verbindung 1 als Lösungsmittel für den UV-Filter Methylbenzylidene-Camphor (20 % bezogen auf das Gemisch mit Verbindung 1). Diese Standardprüfrezeptur dient zur Überprüfung, ob sich der Rezepturbestandteil Tegosoft^{®} TN in Standardprüfrezeptur O/W 1 problemlos gegen eine vergleichbare Menge von Verbindung 1 austauschen lässt, ohne die Eigenschaften der Standardprüfrezeptur grundlegend zu verändern.

| Standardprüfrezeptur O/W | | |
|---|---|---|
| | | % |
| **A** | TEGO^{®} Care 215 | 2,5 |
| | TEGIN^{®} M | 1,0 |
| | TEGO^{®} Alkanol 18 | 2,0 |
| | Verbindung 1 | 11,6 |
| | Methylbenzyliden-campher | 2,9 |
| | (Gesamt. Ölphase 20 %) | |
| | | |
| **B** | Glycerin | 3,0 |
| | Wasser | 76,5 |
| | CA 24^{®} | 0,1 |
| | | |
| **C** | Keltrol F^{®} | 0,4 |

### Standardprüfrezeptur O/W 3:

Mit Verbindung 1 als Lösungsmittel für den UV-Filter Methylbenzylidene-Camphor (30 % bezogen auf das Gemisch mit Verbindung 1). Diese Standardprüfrezeptur dient zur Überprüfung, ob sich der Anteil des UV-Filters Methylbenzyliden-campher in der Standardprüfrezeptur gegenüber Standardprüfrezeptur W/O 2 deutlich erhöhen lässt, ohne die Eigenschaften der Standardprüfrezeptur grundlegend zu verändern.

| Standardprüfrezeptur O/W | | |
|---|---|---|
| | | % |
| **A** | TEGO^{®} Care 215 | 2,5 |
| | TEGIN^{®} M | 1,0 |
| | TEGO^{®} Alkanol 18 | 2,0 |
| | Verbindung 1 | 10,15 |
| | Methylbenzyliden-campher | 4,35 |
| | (Gesamt. Ölphase 20 %) | |
| | | |
| **B** | Glycerin | 3,0 |
| | Wasser | 76,5 |
| | CA 24^{®} | 0,1 |
| | | |
| **C** | Keltrol F^{®} | 0,4 |

Wie aus der Herstellung und Überprüfung der oben angegebenen Rezepturen ersichtlich wurde, kann sowohl der Rezepturbestandteil Tegosoft^{®} TN in Standardprüfrezeptur O/W 1 problemlos gegen eine vergleichbare Menge von Verbindung 1 ausgetauscht werden und auch der Anteil des UV-Filters Methylbenzyliden-campher in der Standardprüfrezeptur deutlich erhöht werden, ohne die Eigenschaften der Standardprüfrezeptur grundlegend zu verändern.

Durch die Herstellung der sechs oben aufgeführten Rezepturen konnte somit gezeigt werden, dass durch die Verwendung der erfindungsgemäßen Verbindungen die Menge an UV-Filter in den Rezepturen um nahezu 100 % erhöht werden konnte.

## Patentansprüche

1. Verwendung von mindestens einer der Verbindungen der Formeln 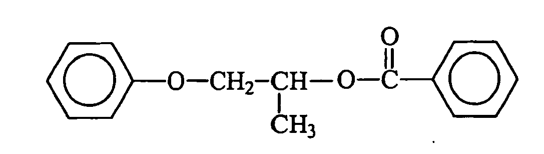 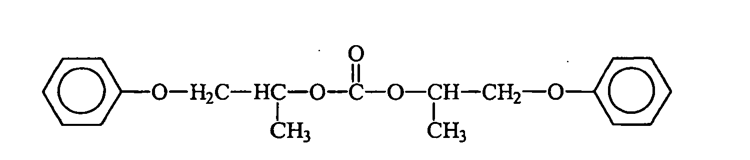 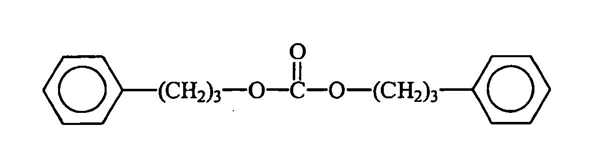 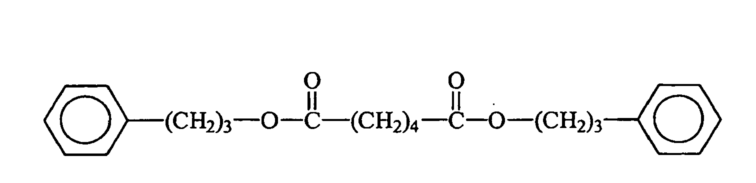 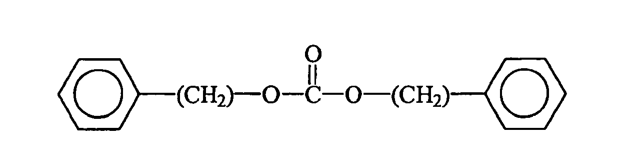 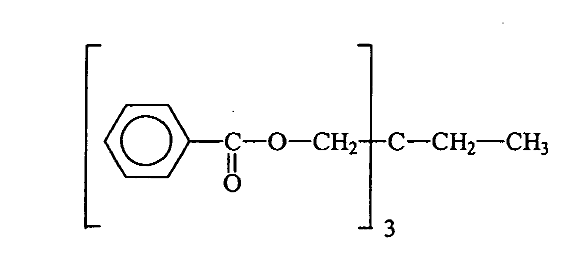 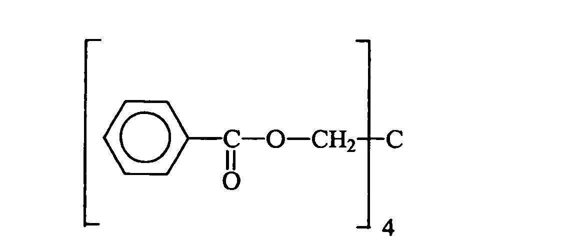 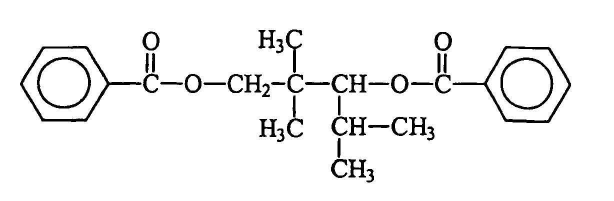 als Lösungsvermittler/Lösungsmittel zur Lösung von organischen W-Filtern.

2. Verwendung der Lösungsvermittler/Lösungsmittel gemäß Anspruch 1 zur Lösung von organischen UV-Filtern für die Herstellung kosmetischer Formulierungen.

3. Kosmetische Formulierungen mit verbessertem Sonnenschutzfaktor enthaltend organische UV-Filter gelöst in den verbesserten Lösungsvermittlern/Lösungsmitteln gemäß Anspruch 1.

## Claims

1. Use of at least one of the compounds of the formulae 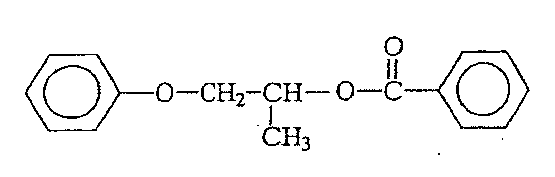 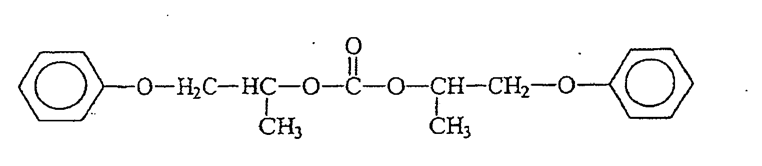 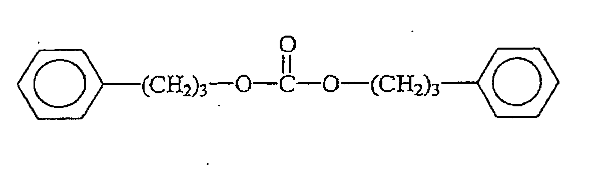 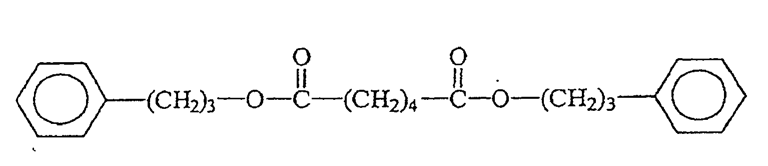 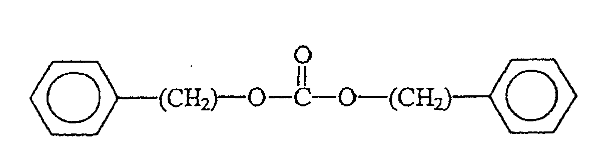 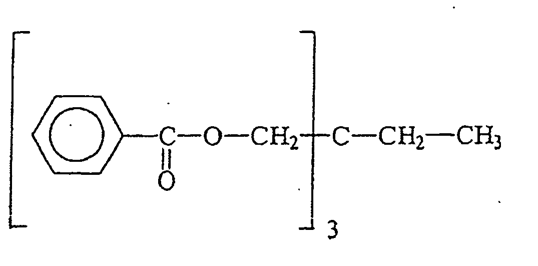 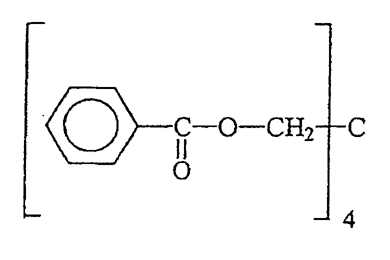 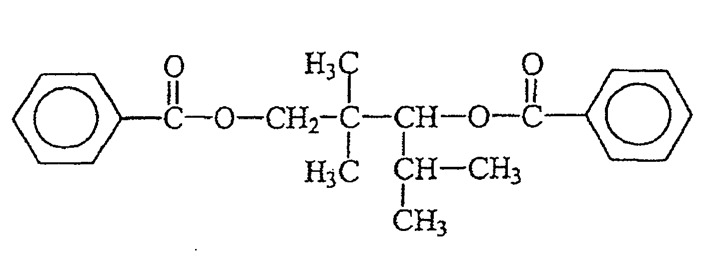 as solubilizing agents/solvents for dissolving organic UV filters.

2. Use of the solubilizing agents/solvents according to Claim 1 for dissolving organic UV filters for the preparation of cosmetic formulations.

3. Cosmetic formulations having improved sun protection factor comprising organic UV filters dissolved in the improved solubilizing agents/solvents according to Claim 1.

## Revendications

1. Utilisation d'au moins un composé des formules 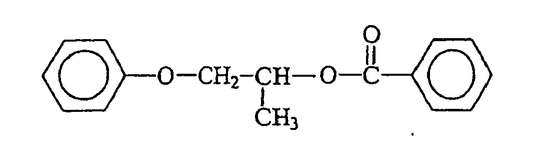 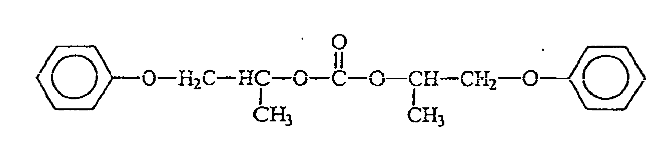 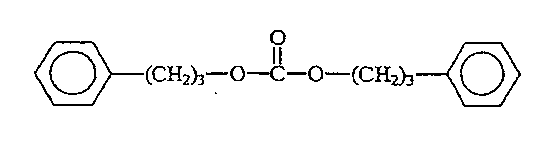 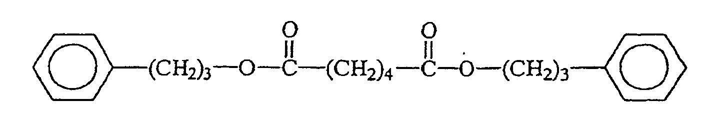 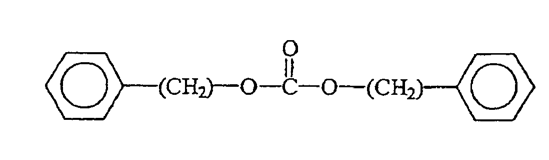 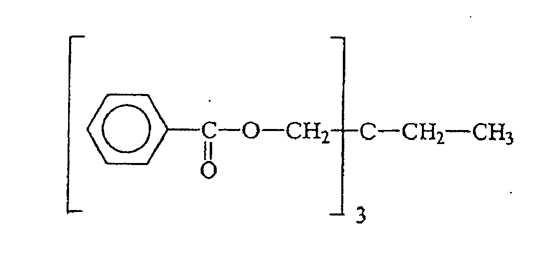 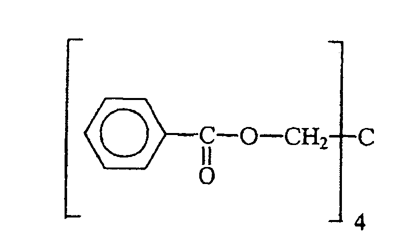 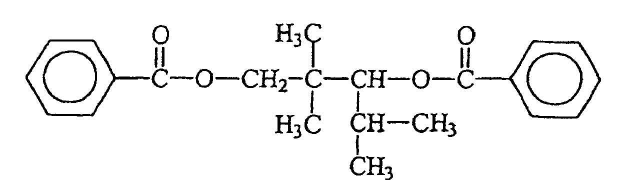 en tant qu'agents de solubilisation / solvants pour la dissolution de filtres UV organiques.

2. Utilisation de l'agent de solubilisation / solvant suivant la revendication 1 pour la dissolution de filtres UV organiques en vue de la préparation de formulations cosmétiques.

3. Formulations cosmétiques présentant un indice de protection solaire amélioré, contenant des filtres UV organiques dissous dans les agents de solubilisation / solvants suivant la revendication 1.
